# EUROPEAN PATENT APPLICATION

(11) **EP 3 721 826 A1**
(43) Date of publication of application: **14.10.2020**
(21) Application number: 19168851.4
(22) Date of filing: 12.04.2019
(51) Int. Cl.: A61B 34/20, A61B 90/00, A61B 17/16, A61B 17/17

(54) **SURGICAL INSTRUMENT SYSTEM FOR TRACKING A RELATIVE POSITION**

(71) Applicant: Medivation AG, 5200 Brugg (CH)
(72) Inventor: Schwägli, Tobias, 4500 Solothurn (CH); Stifter, Jan, 5425 Schneisingen (CH)
(74) Representative: Herrmann, Johanna

(57) **Abstract**

A surgical instrument system comprises an instrument and an optical tracking system (1), the instrument comprising a first part and a movable second part (6) which is movable relative to the first part. A light source (4) is disposed on the first part. The movable second part (6) comprises a reflective surface (5), wherein the movable second part (6) is movable in the direction of the axis of a directional light beam emitted from the light source (4), wherein the position of the movable second part (6) with respect to the first part is measurable by means of the optical tracking system (1) by means of the light beam reflected on the reflective surface (5) of the movable second part (6), wherein the first part can comprise at least three light emitters in a known geometric arrangement, which can form an optical marker (2).

## Description

### Background

The invention is related to a surgical instrument system comprising an instrument and an optical tracking system to determine a relative position of a first part and a movable second part movable relatively to the first part of the instrument. The instrument can in particular comprise a surgical instrument. The invention is used to determine and display the position of the movable second part in relation the first part. Using the reflection of a light beam emitted from a light source, a distance can be determined without requiring an additional marker on the movable second part. The invention can for example be used to determine and display the position of a drill bit relative to a drill guide. The drill depth can be tracked by making use of the reflection of a light beam emitted from a light source without the need for an additional marker on the drill bit.

Tracking of surgical instruments in a surgical instrument system help surgeons to position and align instruments relative to the patient's anatomy. The tracking of the instruments is usually done using optical or electromagnetic tracking systems that allow precise measurements of the position and pose of the instruments and the anatomy. For optical tracking with a stereo or single camera system localization devices can be provided for instance passive reflective markers or active light emitters arranged in a known geometric relation. The position of the instruments can be tracked by shadow imaging whereby the position is calculated by the shadow of the light emitters on an optical sensor as described in EP15192564.1.

### Prior Art

Different methods of drill depth measurement are described in WO2016049467A1. The described methods use resistance, capacitance, optical and/or acoustic sensing features for assisting the drill depth measuring system with determining drill depths and types of tissue being drilled into. The drill depth measurement device is integrated in the drill guide or drill guide handle. A device for measuring the drill depth using an attachment on the drilling machine with a linear sensor is described in patent application document WO2017083989A1 to determine the drill depth.

There are systems determining the drill depth or relative linear movement by using the optical tracking system. US6887247B1 describes use of an optical marker that is attached to the drill bit and not to the drill bit sleeve and moves with the drill bit. In document EP1742583B1, a device is described that measures linear movements of single or multiple optical markers using a stereo camera system. The calculated relative movements are used to calculate forces in a spring engaged system for measuring ligament tension.

US patent applications US6478802B2/US6887245B2 describe the measurement of drill depth using an active marker on the drill guide and an additional marker or active light emitters to determine the relative movement of the drill bit to the drill sleeve.

### Object of the invention

The object of the present invention is to provide a surgical instrument system by means of which the position and pose of an instrument can be precisely determined during an intervention, the instrument functioning as a transmitter.

### Summary of the invention

The object of the invention is achieved by a surgical instrument system according to claim 1. Advantageous embodiments of the surgical instrument system are subject matter of claims 2 to 15.

When the term "for example" is used in the following description, this term refers to embodiments and / or variants, which is not necessarily to be understood as a more preferred application of the teaching of the invention. Similarly, the terms "preferred," "preferable," are understood to refer to an example of a set of embodiments and / or variants, which is not necessarily to be understood as a preferred application of the teaching of the invention. Accordingly, the terms "for example," "preferred," or "preferable," may refer to a plurality of embodiments and / or variants.
The following detailed description contains various embodiments of the inventive device and the inventive method. The description of a particular device of a particular method is to be considered as exemplary only. In the specification and claims, the terms "including," "comprising," "having" are interpreted as "including, but not limited to."
The term "track" or "tracking" is used to measure position or location. The term «navigate» or «navigation» is used for the measurement including its preparation.

A surgical instrument system comprises an instrument and an optical tracking system, the instrument comprising a first part and a movable second part which is movable relative to the first part, wherein a light source is disposed on the first part, wherein the movable second part comprises a reflective surface, wherein the movable second part is movable in the direction of the axis of a directional light beam emitted from the light source, wherein the position of the movable second part with respect to the first part is measurable by means of the optical tracking system by means of the light beam reflected on the reflective surface of the movable second part, wherein the first part can comprise at least three light emitters in a known geometric arrangement, which can form an optical marker.

According to an embodiment the optical tracking system comprises a shadow imaging tracking system. The receiver can be configured as an image sensor. A shadow mask can be arranged on top of the image sensor for measuring the position of the light emitters and the reflected light beam.

According to an embodiment the optical tracking system comprises a receiver, for instance a single camera, a stereo camera system or a plurality of cameras, for capturing the position of the light emitters and the reflected light beam.

In particular, the first part of the instrument can be a drill sleeve and a movable second part can be a drill bit comprising the reflective surface. In particular, the first part of the instrument can be a screw driver housing and a movable second part can be a screw driver comprising the reflective surface.

According to an embodiment the reflective surface is arranged on a removable portion of the movable second part. In particular, the removable portion can be a disposable.

According to an embodiment, a plurality of light sources and a plurality of movable second parts comprising reflective surfaces are provided. In particular, the reflective surface can be of a flat, planar, curved, round, concave or convex shape. The reflective surface can be rotation symmetric with respect to the axis of rotation of the movable second part. The movable second part can comprise a portion disposed with a reflective surface.

According to an embodiment the reflective surface is configured as a diffuse reflective surface. The reflective surface can comprise a polycrystalline material.

According to an embodiment the movable second part is coupled to the first part by a spring element with known properties.

According to an embodiment the movement or the position of the movable second part detected by the receiver is transferable into a digital signal, which is usable as an input dimension for a control unit. The control unit can be integral part of the surgical instrument system and can be used to control the instrument. In particular, the first part can be equipped with an input device to trigger measurement or interaction with the control unit.

To track the localization devices, they previously had to be attached to instruments and the patient anatomy. In various surgical procedures, not only the position of the surgical instrument relative to the body of the patient is of interest, but also the relative movement of a movable second part to a first part of the instrument. By means of a relative measurement by means of a surgical instrument system according to one of the embodiments, for example, the movement or position of a drill bit relative to a drill sleeve can be determined.

The measured value obtainable by the measurement can be converted by a receiver into a digital signal. The digital signal can be used as an input to a control unit of the instrument. The control unit can have an output device, for example a display, to display the measured value or a measured quantity derived from the measured value. For example, on a display not only the drilling trajectory of the drill bit can be displayed, but also the current position of the drill bit and these are displayed in relation to the patient's anatomy.

The patient's anatomy may have been determined by a previous measurement, for example by means of an imaging method, a scanning method or by means of an optical measurement of markers attached to the patient. The measurement of the patient's anatomy can provide measured values which are converted into digital signals and as such can be processed by a processing unit of the control unit or stored as digital data in a memory.

Before the instrument is used, the digital data can be retrievable from a memory of the control unit. They can be converted by the processing unit into image data displayed on the instrument's display or on a display linked to the instrument. For example, the data for patient anatomy and the measured values from the position and orientation measurement can be displayed on or integrated into an image on a portable display, for example by means of virtual reality glasses.

For example, accurately tracking the drilling depth during drilling may be very advantageous in many applications, e.g. for introducing a screw in trauma cases or when attaching pedicle screws in spine surgery. Relative measurements can be used to insert needles or to locate saw blades to perform cuts, to insert screws or other surgical procedures in which a first part of an instrument is moved in a known relationship with another navigated surgical instrument. The information of the instrument position and the position of the movable part can be processed by a computer-aided surgery system by means of a processing unit and displayed on a display unit.

### Brief description of the drawings

The surgical instrument system according to the invention will be described below with reference to a few exemplary embodiments.
- Fig. 1: shows a schematic structure of a surgical instrument system according to a first embodiment of the invention.
- Fig. 2: shows a schematic structure of a surgical instrument system according to a second embodiment of the invention.

### Detailed description of the drawings

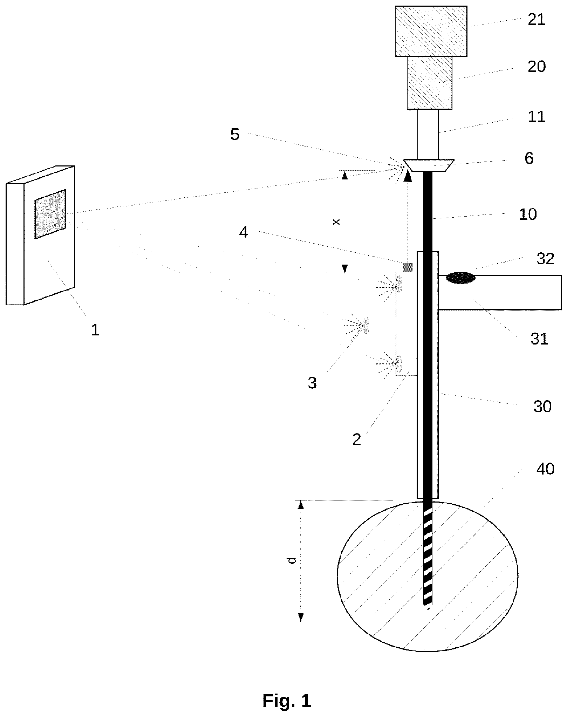

In figure 1 an embodiment of a surgical instrument system according to the invention is shown in combination with an optical tracking system 1, for example, a shadow imaging tracking system. The tracked instrument is according to this embodiment a drill bit comprising a drill guide 30 with a drill guide handle 31 that can be equipped with a button 32 to trigger measurements. The position of this instrument relative to the anatomical structure 40 can be tracked using an optical marker 2 attached to a first part of the instrument. For tracking the pose of a movable second part 6 of the instrument with respect to the anatomical structure 40 another marker can be attached to the anatomical structure. If the camera is a mobile tracking system 1 the camera system itself can be directly attached to the anatomical structure 40. The optical positioning system 1 may comprise a receiver, which may be designed as a camera or a camera system, by means of which reflected light beams emitted by a light source 4 mounted on the first part of the instrument can be received. The light beams are reflected by a reflective surface 5 located on the movable second part 6.

The optical tracking system 1 can be designed as a mobile optical tracking system. For a mobile optical tracking system, the receiver can be attached directly to the anatomical structure 40.

The optical marker 2 is according to this embodiment an active optical marker with at least three light emitters 3 in a known geometric configuration arranged on a marker body. In most applications, infrared LED's are used as light emitters on active optical markers. An active optical marker is to be understood as an optical marker which can emit light. These LED's have a wide beam angle so that they are visible to the optical tracking system at different orientations of the marker body to the receiver. The first part of the instrument 30 or the optical marker 2 comprises a light source 4 for generating a directed light beam with a very narrow beam angle of less than five degrees along the axis of the movable second part 6. In a preferred embodiment, a laser light source or laser LED is used as the light source 4 to generate the directed light beam. In a preferred embodiment light sources are used which emit light beams outside the visible spectrum for example in the infrared range for both the light emitters and the light source for emitting the directed light beam.

The movable second part 6 containing the reflective surface 5 moves along the axis of the light beam, which is emitted by the light source 4. The movable second part 6 containing the reflective surface 5 is attached to the moving part, in this case the drill bit 10. The part 6 containing the reflective surface 5 can be an integral part of the instrument, for instance the drill bit 10 or can be attached to the instrument during the procedure, that means the movable second part can be detachable. For example, the movable second part 2 can be attached temporarily to the instrument by means of a snap device.

The drill bit 10 can have a mating part at a known location so that the position of the reflective surface 5 with respect to the drill bit and especially to the drill tip is known. In another embodiment not shown in Fig. 1 the movable second part 6 forming the reflective surface 5 can also be part of a drill bit adapter 11. The drill bit adapter 11 is configured to attach the drill bit 10 to a drill machine 21. The reflective surface 5 can be on the drill machine 21 itself. For example, the movable second part 6 containing the reflective surface 5 can be placed on the drill chuck 20, in particular, close to the axis of rotation of the drill bit.

In an embodiment, the reflective surface 5 is positioned in an angle with respect to the axis of the directed light beam for reflecting the light in the direction of the receiver, for instance the camera or the camera system.

In the simplest form, a movement of the movable second part 6 can be controlled by means of a key input. The instrument may include a button 32 disposed on the first part which is used as a trigger to move the movable second part 6 to a defined position.

The reflective surface 5 is preferably an ideal diffuse reflector that generates a wide angle reflected light beam. Different materials and surface finishes can be used to generate diffuse reflective surface properties. In an embodiment, a metal surface with a matte surface finish can be used to reflect the directed light beam emitted from the light source. The surface can be made from materials with good diffuse reflectivity, like ceramics or paper. A polycrystalline material may be used. The reflective surface 5 can be coated with a material with a matte surface finish to achieve the desired diffuse optical properties close to a Lambertian reflection. The reflective surface 5 can comprise a surface of a flat, planar, curved, round, convex or concave shape to generate a uniform diffuse reflection with a defined light cone at the point of reflection so to achieve a light scattering.

In an embodiment, the movable second part 6 containing the reflective surface 5 can comprise a transparent material, such as glass or a transparent plastic material, e.g. a polycarbonate, that is designed to reflect an incoming directed light beam similar to a light scattering lens.

In an embodiment the movable second part 6 can be configured as a disposable or can comprise a disposable. The movable second part 6 can comprise a snap on device which is adaptable to different dimensions of the instrument, in particular, different drill diameters. The drill bit can feature for example a notch where the snap on device can be placed. In another embodiment the movable second part 6 is directly attached to the drill bit and forms an integral unit with the drill bit.

If the optical tracking system 1 is used to measure drill bit position the position can also be measured while the drill bit 10 is rotating even at high rotational speeds. For example, the movable second part 6 containing the reflective surface 5 can be rotation symmetric with respect to the drill bit 10. In this configuration, the movable second part 6 does not need to be aligned with the receiver of the optical tracking system 1 or the light source 4.

According to an embodiment, the reflective surface 5 comprises variable reflective properties. For example, more or less light can be reflected in different portions of the reflective surface 5. The reflective surface 5 thus includes at least one reflective surface portion. When the light beam strikes a reflective surface portion, a pulsed reflected light beam is generated. In particular, at least one pulsed reflected light beam per revolution can be generated. The pulsed reflected light beam can be detected by the optical tracking system 1 as a pulse. The period between two pulses can be determined to determine the rotational speed of the drill when a single pulsed light beam is transmitted to the optical tracking system 1 per revolution. Of course, the rotational speed can also be determined if a plurality of pulses per revolution is received by the receiver, if the number of pulses per revolution is known.

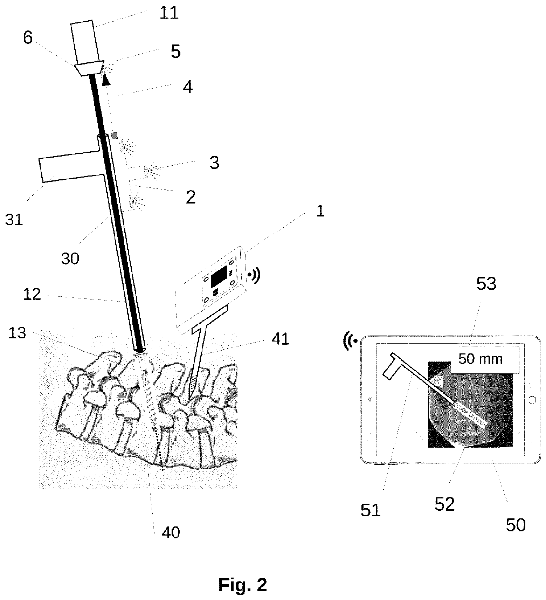

The surgical instrument system according to one of the preceding embodiments can be used to measure drill depth but also to measure depth of a screw insertion wherein the screw driver position relative to the screw guide is measured for example in trauma and spine procedures as shown in fig. 2. The optical tracking system 1 is according to fig. 2 mounted in a known relationship to the first part of the instrument or may also be coupled to the instrument (not shown). The optical tracking system 1 therefore has a frame of reference. The relative movement of the movable second part 6 is determined in the frame of reference of the optical tracking system 1. According to this embodiment, the position of the receiver is known because it is uniquely defined in the frame of reference, therefore no optical markers are needed on the first part. In particular, the position of a camera or a camera system used as a receiver is known. If the first part is a drill sleeve, no optical markers are required on the drill sleeve according to this embodiment, which is not shown in fig. 1.

According to the embodiment shown in fig. 2, the movement of the movable second part 6 to the first part of the instrument can be detected by means of the optical tracking system 1. According to this exemplary embodiment, a receiver is part of the optical tracking system 1. The receiver receives reflected light beams from the reflective surface 5 and the optional optical markers 2 arranged on the first part, if they are designed as active optical markers. The receiver can be designed as a camera or camera system. The optical tracking system 1 can be embodied as a mobile optical tracking system 1, which is attached to the patient's anatomy 40 according to the exemplary embodiment shown in fig. 2.

The optical tracking system 1 thus includes a receiver which detects the movement or the position of the movable second part. The optical tracking system 1 contains a conversion unit, in which the movement or position-determining light signal can be converted into a digital signal which can be used as an input dimension for a control unit 50 of the instrument. The control unit is coupled to or can be coupled to a display unit 53 to display the position 51 of the instrument on the display unit 53, which is shown in fig. 2. For this purpose, the display unit 53 may be configured as a display.

The control unit 50 can, as shown in fig. 2, be wirelessly connected to the receiver of the optical tracking system 1. Thus, the control unit 50 and the receiver are formed as separate components.

According to an embodiment, not shown in the drawings, the control unit and the receiver can be configured as a single component, they may form, for example, a mobile optical tracking system. According to the present embodiment, a screwdriver is used whose position 51 is displayed on the display unit 53. A screw 52 positioned with the screwdriver in the anatomical structure 40 can be displayed on the display unit 53. The dimensions of the screw 52 can be stored, for example, in a memory. The screw can be manually or automatically selected from a database prior to use.

The positioning of the screw on the display unit can be determined, for example, by the rotational axis of the screwdriver determined with the optical tracking system and the position of the anatomical structure. The change in the position of the screwdriver during the procedure is also detected by the optical tracking system. Therefore, the change in the position of the screwdriver can be displayed on the display unit. The position and location of the screw 52 to be positioned in the anatomical structure 40 is thus apparent at any time during the procedure. When the anatomical structure 40 has been previously detected by imaging techniques, the position of the screw 52 in the anatomical structure can be precisely displayed at any time of the procedure.

A further advantage of the use of a surgical instrument system according to the invention is that the position of a tracked instrument can be precisely visualized, thus increasing the precision of the procedure and ensuring that there are no complications during the procedure, in particular, because details of the anatomical structure can be displayed in an image, such as a sectional image, and thus, the further course of the procedure on the basis of this pictorial representation is visible and predictable with increased precision.

According to an embodiment, the surgical instrument system can include a plurality of instruments. The relative movement of two or more instruments with corresponding movable second parts can be determined by using multiple light sources. Each light source transmits light beams to corresponding reflective surfaces located on the respective movable second parts of the respective instruments. The reflected light beams are detected by the receiver and the position of the instruments are determined by means of the optical tracking system.

According to an embodiment, the invention can be used to measure forces acting on the movable second part of the instrument. If a spring element is arranged between the first part and the movable second part and the spring properties are known based on the measured distance, the force applied to the spring element can be calculated. For example, such an instrument may be used to measure forces for the ligament tensioner in a knee replacement surgery. According to a further application the movable second part is used to measure the distance or depth, wherein the first part engages the spring element and is used to measure the applied force.

According to each of the preceding embodiments, the movement of the movable second part to the first part of the instrument can be detected by means of the optical tracking system 1. The movement detected by the receiver or the position of the movable second part can be converted by a conversion unit into a digital signal, which can be used as an input dimension for a control unit of the instrument. The control unit may be coupled to a display unit or coupled to indicate the position of the instrument on the display unit, which is shown in fig. 2.

According to an embodiment, the movement of the instrument is detected by the receiver as an analog input signal for the control unit, wherein a value in the control unit can be set or changed with the measured displacement of the movable second part. In particular, implant sizes, screw lengths can be set by the operator without the need for an additional input unit.

According to an embodiment the instrument is equipped with at least one button 32 to trigger measurements or interactions with the control unit. The control unit can be a part of a navigation system. For example, the navigation system can provide a switch element. A registration of the relative position of the movable second part with respect to the first part can be triggered manually or automatically by the switch element. The signal generated by the manipulation of the switch element, e.g. by pressing the button, can be transmitted using an optical signal or using wireless communication protocol to the tracking system 1 or navigation system.

The proposed surgical instrument system has several advantages compared to existing solutions. The position of an instrument, for example of a drill bit or a screw driver can be tracked without the need for an additional active marker attached to the instrument, e.g. the drill bit or drilling machine. By using the reflection of a directed light beam emitted from the light source, the movable second part 6 comprising the reflective surface 5 on the instrument can act as a light emitter to be tracked by an optical tracking system 1. The reflective surface can be part of the instrument itself or can be attached to it.

A big advantage is that the proposed solution also works when the instrument is rotating. According to this embodiment, the reflective surface 5 of the movable second part 6 is designed as a rotation symmetric reflective surface. The invention does not require the alignment of a marker on the instrument with the receiver.

The proposed invention can be used with a shadow imaging system where the light emitters cast a shadow through a defined pattern on the receiver which is configured as a sensor comprising an optical sensor surface. Based on the shadow on the sensor the direction of the light emitters can be determined and the 6D position of the tracked instrument and the relative movement the light beam reflected from the reflective surface can be determined. The receiver can comprise a single, stereo or multi camera system instead of a sensor, which receives the light beams from light emitters.

It will be apparent to those skilled in the art that many more variations are possible in addition to the described embodiments without departing from the inventive concept. The object of the invention is thus not limited by the foregoing description and is determined by the scope of protection defined by the claims. The widest possible reading of the claims is decisive for the interpretation of the claims or the description. In particular, the terms "contain" or "include" are to be interpreted as referring to elements, components or steps in a non-exclusive sense, to indicate that the elements, components or steps may be present or used and can be combined with other elements, components, or steps that are not explicitly mentioned. When the claims refer to an element or component from a group which may consist of A, B, C to N elements or components, that formulation should be interpreted as requiring only a single element of that group, rather than a combination of A and N, B and N or any other combination of two or more elements or components of this group.

## Claims

1. A surgical instrument system comprising an instrument and an optical tracking system, the instrument comprising a first part and a movable second part which is movable relative to the first part, wherein a light source is disposed on the first part, wherein the movable second part comprises a reflective surface, wherein the movable second part is movable in the direction of the axis of a directional light beam emitted from the light source, wherein the position of the movable second part with respect to the first part is measurable by means of the optical tracking system by means of the light beam reflected on the reflective surface of the movable second part, wherein the first part can comprise at least three light emitters in a known geometric arrangement, which can form an optical marker.

2. The surgical instrument system of claim 1, wherein the optical tracking system comprises a shadow imaging tracking system.

3. The surgical instrument system according to one of the preceding claims, wherein the optical tracking system comprises a receiver, for instance a single camera, a stereo camera system or a plurality of cameras, for capturing the position of the light emitters and the reflected light beam.

4. The surgical instrument system according to one of the preceding claims, wherein the first part is a drill sleeve and a movable second part is a drill bit comprising the reflective surface.

5. The surgical instrument system according to one of the preceding claims, wherein the reflective surface is arranged on a removable portion of the movable second part.

6. The surgical instrument system according to claim 5 wherein the removable portion is a disposable.

7. The surgical instrument system according to one of the preceding claims, wherein a plurality of light sources and a plurality of movable second parts comprising reflective surfaces are provided.

8. The surgical instrument system according to one of the preceding claims, wherein the reflective surface is of a flat, planar, curved, round, concave or convex shape.

9. The surgical instrument system according to one of the preceding claims, wherein the reflective surface is rotation symmetric with respect to the axis of rotation of the movable second part.

10. The surgical instrument system according to one of the preceding claims, wherein the movable second part comprises a portion disposed with a reflective surface.

11. The surgical instrument system according to one of the preceding claims, wherein the reflective surface is configured as a diffuse reflective surface.

12. The surgical instrument system according to one of the preceding claims, wherein the reflective surface comprises a polycrystalline material.

13. The surgical instrument system according to one of the preceding claims wherein the movable second part is coupled to the first part by a spring element with known properties.

14. The surgical instrument system according to one of the preceding claims 3 to 13 wherein the movement or the position of the movable second part detected by the receiver is transferable into a digital signal, which is usable as an input dimension for a control unit.

15. The surgical instrument system according to claim 15 wherein the first part is equipped with an input device to trigger measurement or interaction with the control unit.
